# EUROPÄISCHE PATENTANMELDUNG

(11) **EP 2 149 356 A2**
(43) Veröffentlichungstag der Anmeldung: **03.02.2010**
(21) Anmeldenummer: 09009963.1
(22) Anmeldetag: 01.08.2009
(51) Int. Cl.: A61F 2/84

(54) **Verfahren und Vorrichtung zum Beladen eines Stentapplikators**

(30) Priorität: 02.08.2008 DE 102008036205
(71) Anmelder: Karl Storz GmbH & Co. KG, 78532 Tuttlingen (DE)
(72) Erfinder: Pilz, Kevin, 78532 Tuttlingen (DE); Hermle, Rainer, 78559 Gosheim (DE)
(74) Vertreter: Hofmeister, Frank

(57) **Zusammenfassung**

Die Erfindung betrifft ein Verfahren und eine Vorrichtung zum Beladen eines Stentapplikators (2). Ein Verfahren zum Beladen eines Stentapplikators (2), das bei einfacher Handhabung und ohne Verletzungsrisiko das Beladen eines Stentapplikators (2) ermöglicht, ist erfindungsgemäß gekennzeichnet durch die Verfahrensschritte:
a) Ausüben einer seitlichen Druckkraft auf den Stent (13) zur Verkleinerung dessen Durchmessers,
b) Einfügen des zusammengedrückten Stents (13) in eine eine ZylinderKolben-Anordnung aufweisende Beladevorrichtung (1),
c) zumindest teilweises Einführen der Beladevorrichtung (1) in den Stentapplikator (2) und
d) Beladen des Stentapplikators (2) mit dem Stent (13) durch Verschieben der Bauteile Zylinder (3) und Kolben (4) der Beladevorrichtung (2) relativ zueinander.

## Beschreibung

Die Erfindung betrifft ein Verfahren und eine Vorrichtung zum Beladen eines Stentapplikators.

Unter Stents versteht man in der Chirurgie endoskopisch platzierte Röhrchen zur Überbrückung oder Drainage bei Stenosen, Strikturen und Tumoren. Bei den Stents handelt es sich um aus verschiedenen körperverträglichen Materialien hergestellte rohrförmige Prothesen, die nach der endoskopischen oder radiologischen Implantation dazu dienen, beispielsweise bei tumorbedingter Stenose und Obstruktion oder bei arteriosklerotisch bedingter kurzstreckiger Gefäßstenose, das Lumen eines Hohlorgans zu überbrücken oder zu erhalten.

Aus der DE 102 49 927 B3 ist beispielsweise eine Vorrichtung zum Komprimieren rohrförmiger Endoprothesen sowie zum Einführen einer komprimierten Endoprothese in ein Applikationsrohr bekannt. Diese bekannte Vorrichtung dient dazu, aus einem faltbaren elastischen Material bestehende Stents durch Ausbildung einer Längsfaltung zu komprimieren und die solchermaßen komprimierten Stents nachfolgend mittels eines Ausstoßwerkzeugs in ein Applikationsrohr zu überführen.

Neben den elastischen und meist aus einem Kunststoffmaterial bestehenden Stents gibt es jedoch auch Drahtstents, die meist aus einem Drahtgeflecht gefertigt sind. Diese Drahtstents lassen sich zwar durch seitliches Drücken im Durchmesser reduzieren, jedoch ist das Ausbilden einer sich reversibel wieder zurückbildenden Längsfaltung in der Regel aufgrund der Materialsteifigkeit nicht möglich. Aus diesem Grund werden die Draht- und Drahtgeflechtstents in der Praxis von Hand in die Stentapplikatoren hineingeschoben. Dieses manuelle Beladen ist sehr aufwendig, da der Stent den Durchmesser des Applikators meist überschreitet und somit beim Hineinschieben verklemmen kann. Darüber hinaus stehen an den Enden der Drahtgeflechtstents zumeist spitze Drähte hervor, an denen sich der Bediener beim Einführen des Stents in den Stentapplikator leicht verletzen kann.

Davon ausgehend liegt der Erfindung die **Aufgabe** zugrunde, ein Verfahren und eine Vorrichtung zum Beladen eines Stentapplikators zu schaffen, die bei einfacher Handhabung und ohne Verletzungsrisiko das Beladen eines Stentapplikators ermöglichen.

Die verfahrensmäßige **Lösung** dieser Aufgabenstellung ist gekennzeichnet durch die Verfahrensschritte:
a) Ausüben einer seitlichen Druckkraft auf den Stent zur Verkleinerung dessen Durchmessers,
b) Einfügen des zusammengedrückten Stents in eine eine Zylinder-Kolben-Anordnung aufweisende Beladevorrichtung,
c) zumindest teilweises Einführen der Beladevorrichtung in den Stentapplikator und
d) Beladen des Stentapplikators mit dem Stent durch Verschieben der Bauteile Zylinder und Kolben der Beladevorrichtung relativ zueinander.

Mit dem erfindungsgemäßen Verfahren ist es möglich, auch Drahtstents ohne Verletzungsrisiko für den Bediener in einen Stentapplikator zu überführen. Lediglich das Ausüben einer seitlichen Druckkraft auf den Stent zur Verkleinerung dessen Durchmessers im Verfahrensschritt a) erfolgt bei einer Verfahrensvariante manuell, jedoch ist diese manuelle Betätigung nicht mit einem Verletzungsrisiko für den Ausführenden verbunden, da der Stent von den Seiten her zusammengedrückt wird, nicht aber über die mit den spitzen Drahtenden versehenen Enden des Stents.

Gemäß einer alternativen Ausführungsform des Verfahrensschritts a) wird erfindungsgemäß vorgeschlagen, dass die seitliche Druckkraft auf den Stent mittels einer Pressvorrichtung ausgeübt wird.

Die Pressvorrichtung zum Ausüben einer seitlichen Druckkraft auf den Stent zur Verkleinerung dessen Durchmessers weist gemäß einer praktischen Ausführungsform der Erfindung mindestens ein den Umfang des Stents zumindest teilweise umgreifendes Druckmittel auf, über das die seitliche Druckkraft auf den Stent übertragen wird.

Gemäß einer ersten Ausführungsform zur Ausbildung der Pressvorrichtung wird vorgeschlagen, dass das mindestens eine Druckmittel als Maulteile eines zangenartigen Werkzeugs ausgebildet ist.

Gemäß einer zweiten Ausführungsform zur Ausbildung der Pressvorrichtung wird vorgeschlagen, dass das mindestens eine Druckmittel als den Stent umfänglich umschlingendes und zusammenziehbares biegsames Band, wie beispielsweise ein an sich bekannter Kabelbinder, ausgebildet ist.

Das eigentliche Einsetzen des im Durchmesser reduzierten Stents in die Beladevorrichtung erfolgt erfindungsgemäß über einen seitlichen Schlitz im Zylinder der Beladevorrichtung. Diese art der Einbringung des Stents in die Beladevorrichtung ist vorteilhaft, da es keiner Druckausübung auf die mit den spitzen Drahtenden versehenen Enden des Stents bedarf.

Zum Überführen des Stents in den Stentapplikator wird gemäß einer ersten Ausführungsform der Erfindung vorgeschlagen, dass im Verfahrensschritt d) zum Entleeren der Beladevorrichtung der Kolben festgehalten und der Zylinder vom Stentapplikator fort bewegt wird. Diese Art der Entleerung der Beladevorrichtung hat den Vorteil, dass der Stent nicht innerhalb des Applikators verschoben werden muss.Gemäß einer zweiten Ausführungsform des erfindungsgemäßen Verfahrens wird vorgeschlagen, dass im Verfahrensschritt d) zum Entleeren der Beladevorrichtung der Zylinder festgehalten und der Kolben zum Stentapplikator hin bewegt wird. Bei dieser alternativen Ausführungsform muss der Zylinder nicht so weit in den Stentapplikator eingeführt werden, da das eigentliche Einsetzen des Stents in den Applikator durch Einschieben über den Kolben erfolgt.

Die vorrichtungsmäßige **Lösung** dieser Aufgabenstellung ist gekennzeichnet durch eine Zylinder-Kolben-Anordnung mit einem zumindest teilweise in Längsrichtung geschlitzten Zylinder und einem verschiebbar in dem Zylinder gelagerten Kolben.

Mit der erfindungsgemäßen Vorrichtung ist es erstmalig möglich, auch Drahtstents weitestgehend ohne manuelle Handhabung und somit ohne Verletzungsrisiko für den Bediener in einen Stentapplikator zu überführen.

Gemäß einer bevorzugten Ausführungsform der Erfindung wird vorgeschlagen, dass der Zylinder als in Längsrichtung geschlitztes Rohr mit einer am proximalen Ende angeordneten Handhabe ausgebildet ist, wobei der Schlitz bis zum distalen Ende des Zylinderrohres reicht. Diese Art der Ausbildung des Zylinders ermöglicht bei einfacher und kostengünstiger Herstellung eine leichte Handhabung der Vorrichtung.

Die Handhabe zum Bedienen des Zylinders ist erfindungsgemäß vorteilhafterweise als auf das proximale Ende des Zylinderrohres aufgesetzte Scheibe ausgebildet, wobei die scheibenförmige Ausbildung der Handhabe ein gutes und sicheres Ergreifen der Handhabe gewährleistet.

Weiterhin wird mit der Erfindung vorgeschlagen, dass der Kolben als im Wesentlichen formschlüssig im Zylinderrohr gelagerter und in Längsrichtung des Zylinderrohres verschiebbarer stangenförmiger Stempel mit einer am proximalen Ende angeordneten Handhabe ausgebildet ist. Während das Zylinderrohr als Aufnahme für den Stent dient, wirkt der als stangenförmiger Stempel ausgebildete Kolben als Auswerfwerkzeug zum Entleeren des Zylinderrohres und Überführen des Stents in den Applikator.

Gemäß einer praktischen Ausführungsform der Erfindung wird vorgeschlagen, dass die Handhabe als auf das Zylinderrohr aufgesetzte Scheibe ausgebildet ist, die im Bereich des im Zylinderrohr ausgebildeten Schlitzes über einen Steg mit dem Kolben verbunden ist.

Schließlich wird mit der Erfindung vorgeschlagen, dass die Stents vorzugsweise als Drahtgeflechtstents ausgebildet sind. Die erfindungsgemäße Ladevorrichtung ist insbesondere für jede Art von Drahtstents geeignet, da es zu keinem manuellen Kontakt mit den spitzen und ein hohes Verletzungsrisiko aufweisenden Stentenden kommt.

Weitere Merkmale und Vorteile der Erfindung ergeben sich anhand der zugehörigen Zeichnung, in der ein Ausführungsbeispiel einer erfindungsgemäßen Vorrichtung zum Beladen eines Stentapplikators nur beispielhaft dargestellt ist, ohne die Erfindung auf dieses Ausführungsbeispiel zu beschränken. In der Zeichnung zeigt:
- Fig. 1: eine perspektivische Seitenansicht einer erfindungsgemäßen Vorrichtung zum Beladen eines Stentapplikators im demontierten Zustand;
- Fig. 2: eine Draufsicht auf die Vorrichtung gemäß Fig. 1, jedoch die Vorrichtung zusammengesetzt und mit eingelegtem Stent darstellend;
- Fig. 3a: eine Seitenansicht eines Stents im Ausgangszustand und
- Fig. 3b: eine Seitenansicht des durch Ausüben einer seitlichen Druckkraft im Durchmesser reduzierten Stents gemäß Fig. 3a.

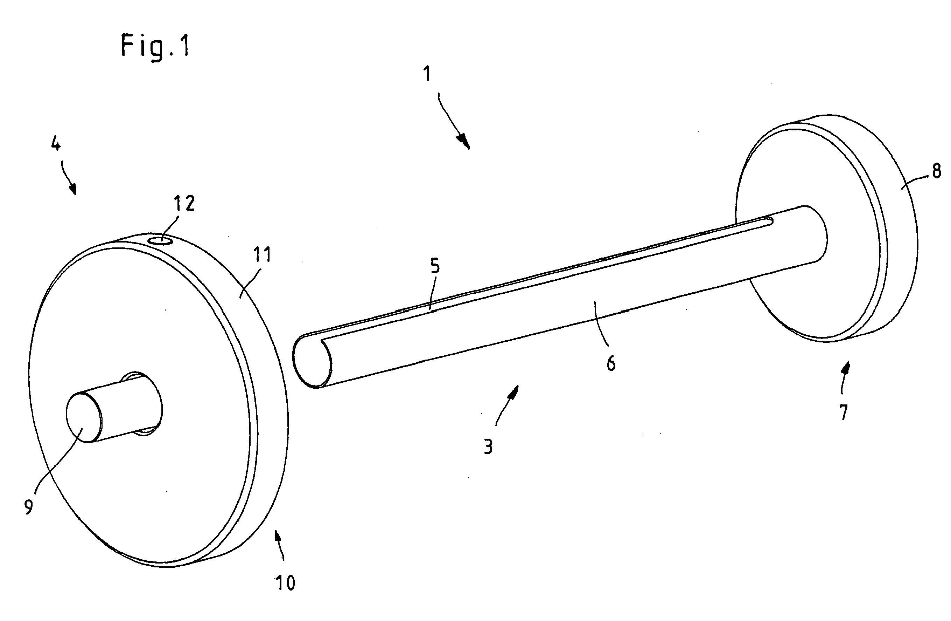

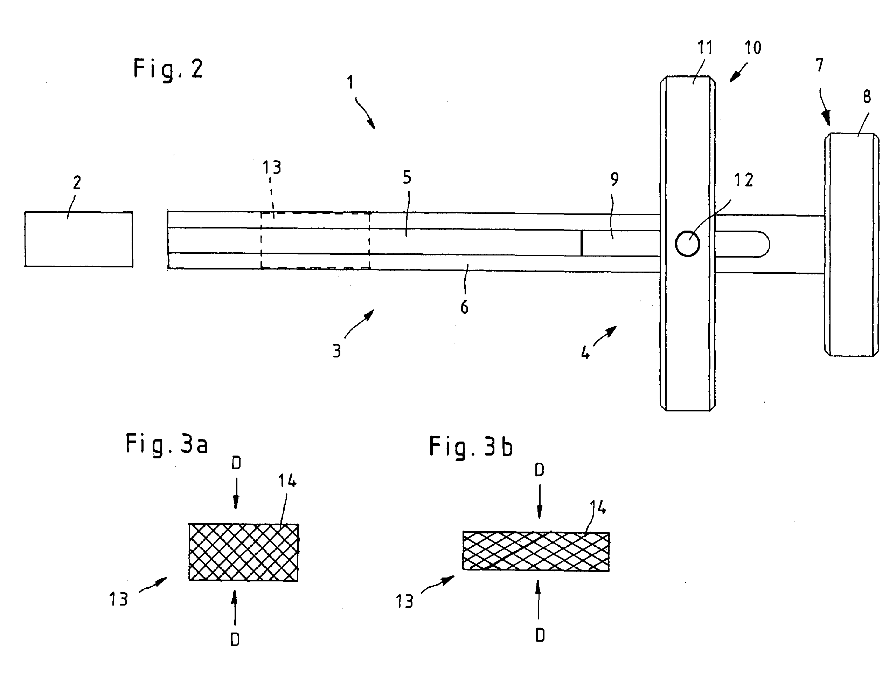

Die in den Abbildungen Fig. 1 und 2 dargestellte Beladevorrichtung 1 zum Beladen eines Stentapplikators 2 besteht im Wesentlichen aus einer einen Zylinder 3 und einen Kolben 4 umfassenden Zylinder-Kolben-Anordnung.

Wie aus Fig. 1 und 2 ersichtlich, ist der Zylinder 3 als zumindest teilweise mit einem Längsschlitz 5 versehenes Rohr 6 ausgebildet, wobei der Längsschlitz 5 bis zum distalen Ende des Zylinderrohres 6 reicht. Am proximalen Ende des Zylinderrohres 6 ist eine Handhabe 7 angeordnet, die bei der dargestellten Ausführungsform als auf das proximale Ende des Zylinderrohres 6 aufgesetzte Scheibe 8 ausgebildet ist.

Der Kolben 4 ist als stangenförmiger Stempel 9 ausgebildet, der im montierten Zustand der Beladevorrichtung 1 im Wesentlichen formschlüssig und in Längsrichtung des Zylinderrohres 6 verschiebbar im Zylinderrohr 6 gelagerter ist. Am proximalen Ende des Kolbens ist eine Handhabe 10 angeordnet, die bei der dargestellten Ausführungsform als auf das Zylinderrohr 6 aufgesetzte Scheibe 11 ausgebildet ist, die im Bereich des im Zylinderrohr 6 ausgebildeten Schlitzes 5 über einen Steg 12 mit dem Stempel 9 verbunden ist. Wie aus Fig. 2 ersichtlich, dient der Steg 12 gleichzeitig als verdrehsichere Führung beim Verschieben des Stempels 9 entlang dem Schlitz 5 des Zylinderrohres 6.

Das Beladen eines Stentapplikators 2 mit einem Stent 13 mittels der zuvor beschriebenen Beladevorrichtung 1 geschieht wie folgt:

Ausgehend von dem in Fig. 1 dargestellten demontierten Zustand der Beladevorrichtung 1 wird zunächst die Zylinder-Kolben-Anordnung durch Einsetzen des Stempels 9 des Kolbens 4 in das Zylinderrohr 6 komplettiert. Der den Stempel 9 mit der Handhabe 10 verbindende Steg 12 erleichtert dabei das verkantungsfreie Einschieben des Stempels 9 in das Zylinderrohr 6.

Im in Fig. 2 dargestellten montierten und einsatzbereiten Zustand der Beladevorrichtung 1 umgibt das Zylinderrohr 6 den Stempel 9 bis auf den Bereich des Schlitzes 5 koaxial und im Wesentlichen formschlüssig.

Das Einsetzen des Stents 13 in den Zylinder 3 der Beladevorrichtung 1 erfolgt über den im Zylinderrohr 6 ausgebildeten Schlitz 5. Hierzu wird der Stent 13, wie in Fig. 3a und 3b dargestellt, durch Ausüben einer seitlichen Druckkraft D im Durchmesser so weit reduziert, bis der Stent 13 durch den Schlitz 5 in das Zylinderrohr 6 einsetzbar ist. Fig. 2 zeigt in der Draufsicht die Beladevorrichtung 1 mit einem im Zylinderrohr 6 angeordneten Stent 13.

Gerade bei Drahtgeflechtstents bewirkt das Ausüben der seitlichen Druckkraft D auf den Stent 13 aufgrund der gegenseitig verschiebbaren Drähte 14 des Drahtgeflechts eine Reduzierung des Außendurchmessers bei gleichzeitiger Streckung des Stents 13, wie dies in Fig. 3a und 3b exemplarisch vergrößert dargestellt ist, wobei Fig. 3a den Ausgangszustand des Stents 13 zeigt und Fig. 3b den durch Ausüben der seitlichen Druckkraft D im Durchmesser reduzierten, aber gleichzeitig in der Länge gestreckten Stent 13 zeigt.

Das Ausüben der seitlichen Druckkraft D auf den Stent 13 erfolgt in der Regel manuell, jedoch ist es auch möglich, zur Ausübung der seitlichen Druckkraft D auf den Stent 13 eine Pressvorrichtung, beispielsweise in Form einer Zange oder eines Bandes, zu verwenden. Bei einem solchen als Pressvorrichtung verwendbaren Band kann es sich beispielsweise um handelsübliche Kabelbinder handeln.

Nachfolgend wird das Zylinderrohr 6 der Beladevorrichtung 1 mit dem distalen Ende voran in den Stentapplikator 2 eingeschoben, bis der Stent 13 vollständige Aufnahme im Stentapplikator 2 findet.

Zum Überführen des Stents 13 in den Stentapplikator 2 wird nun der Kolben 4 so weit zum Stentapplikator 2 hin verschoben, bis das distale Ende des Stempels 8 am proximalen Ende des noch im Zylinderrohr 6 befindlichen Stents 13 anliegt. Das eigentliche Entleeren der Beladevorrichtung 1 bzw. Beladen des Stentapplikators 2 erfolgt im letzten Verfahrensschritt. Dabei wird der Kolben 4 über die Handhabe 10 in seiner Lage zum Stentapplikator 2 fixiert und der Zylinder 3 über die Handhabe 7 vom Stentapplikator 2 fort aus dem Stentapplikator 2 herausgezogen.

Diese Relativbewegung zwischen dem Kolben 3 und dem Zylinder 4 bewirkt, dass der Stent 13 durch den Stempel 9 des Kolbens 4 beim Zurückziehen des Zylinders 3 in seiner Position im Stentapplikator 2 gehalten wird und nach dem vollständigen Zurückziehen des Zylinders 3 vollständig und bereit zur nachfolgenden chirurgischen Platzierung im Stentapplikator 2 plaziert ist.

Alternativ zu der zuvor beschriebenen Art der Entleerung der Beladevorrichtung 1 bzw. Beladung des Stentapplikators 2 ist es auch möglich, den im Zylinderrohr 6 angeordneten Stent 13 aktiv über den Stempel 9 des Kolbens 4 aus dem Zylinderrohr 6 herausgeschoben wird.

Bei dieser Ausführungsform des Beladeverfahrens wird das Zylinderrohr 6 nur ein kurzes Stück in den Stentapplikator 2 eingeführt und anschließend unter Fixierung des Zylinders 3 in dieser Stellung im Stentapplikator 2 der Kolben 4 nach vorne zum Stentapplikator 2 hin verschoben, bis der Stempel 9 den Stent 13 vollständig aus dem Zylinderrohr 6 heraus und in den Stentapplikator 2 hinein gedrückt hat.

Die voranstehend beschriebene Beladevorrichtung 1 ist insbesondere zum Beladen eines Stentapplikators 2 mit Drahtgeflechtstents 13 vorteilhaft, da das eigentliche Einführen des Stents 13 in den Stentapplikator 2 ohne manuelles Zutun erfolgt, wodurch ein Verletzungsrisiko für den Benutzer der Beladevorrichtung 1 durch die scharfen und spitzen Enden der Drahtgeflechtstents 13 ausgeschlossen werden kann.

### Bezugszeichenliste

- 1: Beladevorrichtung
- 2: Stentapplikator
- 3: Zylinder
- 4: Kolben
- 5: Längsschlitz/Schlitz
- 6: Rohr/Zylinderrohr
- 7: Handhabe
- 8: Scheibe
- 9: Stempel
- 10: Handhabe
- 11: Scheibe
- 12: Steg
- 13: Stent
- 14: Draht

- D: Druckkraft

## Patentansprüche

1. Verfahren zum Beladen eines Stentapplikators (2),
**gekennzeichnet durch**
die Verfahrensschritte:
a) Ausüben einer seitlichen Druckkraft auf den Stent (13) zur Verkleinerung dessen Durchmessers,
b) Einfügen des zusammengedrückten Stents (13) in eine eine Zylinder-Kolben-Anordnung aufweisende Beladevorrichtung (1),
c) zumindest teilweises Einführen der Beladevorrichtung (1) in den Stentapplikator (2) und
d) Beladen des Stentapplikators (2) mit dem Stent (13) **durch** Verschieben der Bauteile Zylinder (3) und Kolben (4) der Beladevorrichtung (1) relativ zueinander.

2. Verfahren nach Anspruch 1, **dadurch gekennzeichnet, dass** im Verfahrensschritt a) die seitliche Druckkraft auf den Stent (13) manuell ausgeübt wird.

3. Verfahren nach Anspruch 1, **dadurch gekennzeichnet, dass** im Verfahrensschritt a) die seitliche Druckkraft auf den Stent (13) mittels einer Pressvorrichtung ausgeübt wird.

4. Verfahren nach einem der Ansprüche 1 bis 3, **dadurch gekennzeichnet, dass** der Stent (13) im Verfahrensschritt b) über einen seitlichen Schlitz (5) in die Beladevorrichtung (1) eingesetzt wird.

5. Verfahren nach einem der Ansprüche 1 bis 4, **dadurch gekennzeichnet, dass** im Verfahrensschritt d) zum Entleeren der Beladevorrichtung (1) der Kolben (4) festgehalten und der Zylinder (3) vom Stentapplikator (2) fort bewegt wird.

6. Verfahren nach einem der Ansprüche 1 bis 4, **dadurch gekennzeichnet, dass** im Verfahrensschritt d) zum Entleeren der Beladevorrichtung (1) der Zylinder (3) festgehalten und der Kolben (4) zum Stentapplikator (2) hin bewegt wird.

7. Vorrichtung zum Beladen eines Stentapplikators (2),
**gekennzeichnet durch**
eine Zylinder-Kolben-Anordnung mit einem zumindest teilweise in Längsrichtung geschlitzten Zylinder (3) und einem verschiebbar in dem Zylinder (3) gelagerten Kolben (4).

8. Vorrichtung nach Anspruch 7, **dadurch gekennzeichnet, dass** der Zylinder (3) als in Längsrichtung geschlitztes Rohr (6) mit einer am proximalen Ende angeordneten Handhabe (7) ausgebildet ist, wobei der Schlitz (5) bis zum distalen Ende des Zylinderrohres (6) reicht.

9. Vorrichtung nach Anspruch 8, **dadurch gekennzeichnet, dass** die Handhabe (7) als auf das proximale Ende des Zylinderrohres (6) aufgesetzte Scheibe (8) ausgebildet ist.

10. Vorrichtung nach einem der Ansprüche 7 bis 9, **dadurch gekennzeichnet, dass** der Kolben (4) als im Wesentlichen formschlüssig im Zylinder (3) gelagerter und in Längsrichtung des Zylinders (3) verschiebbarer stangenförmiger Stempel (9) mit einer am proximalen Ende angeordneten Handhabe (10) ausgebildet ist.

11. Vorrichtung nach Anspruch 10, **dadurch gekennzeichnet, dass** die Handhabe (10) als auf das Zylinderrohr (6) aufgesetzte Scheibe (11) ausgebildet ist, die im Bereich des im Zylinderrohr (6) ausgebildeten Schlitzes (5) über einen Steg (12) mit dem Kolben (4) verbunden ist.

12. Vorrichtung nach einem der Ansprüche 7 bis 11, **dadurch gekennzeichnet, dass** die Stents (13) vorzugsweise als Drahtgeflechtstents ausgebildet sind.

13. Pressvorrichtung zum Ausüben einer seitlichen Druckkraft auf einen Stent (13) zur Verkleinerung dessen Durchmessers,
**gekennzeichnet durch**
mindestens ein den Umfang des Stents (13) zumindest teilweise umgreifendes Druckmittel.

14. Pressvorrichtung nach Anspruch 13, **dadurch gekennzeichnet, dass** das mindestens eine Druckmittel als Maulteile eines zangenartiges Werkzeugs ausgebildet ist.

15. Pressvorrichtung nach Anspruch 13, **dadurch gekennzeichnet, dass** das mindestens eine Druckmittel als den Stent (13) umfänglich umschlingendes und zusammenziehbares biegsames Band ausgebildet ist.
